(19) Europäisches Patentamt
European Patent Office
Office européen des brevets

(11) Publication number: **0 249 509 B1**

(12) **EUROPEAN PATENT SPECIFICATION**

(45) Date of publication of patent specification: **21.11.91**

(51) Int. Cl.⁵: **A61B 5/08**, A61B 5/113, G01D 5/14

(21) Application number: **87305303.7**

(22) Date of filing: **15.06.87**

(54) Displacement sensor.

(30) Priority: **13.06.86 GB 8614504**

(43) Date of publication of application:
**16.12.87 Bulletin 87/51**

(45) Publication of the grant of the patent:
**21.11.91 Bulletin 91/47**

(84) Designated Contracting States:
**DE ES FR GB IT NL**

(56) References cited:
FR-A- 2 353 268
GB-A- 2 176 610

PROCEEDING OF NAT. ELECTR. CONF., vol. 33, 29th/31st October 1979, pages 280-285; M. NALECZ et al.: "Application of Hall effect transducers to measurements of mechanical phenomena in cardiovascular system"

MICROPROCESSORS AND MICROSYSTEMS, vol.8, no. 8, October 1984, pages 424-429, Whitstable, GB; C. SMITH "Interfaces and programs for classroom experiments in biology"

(73) Proprietor: **DENSA LIMITED**
**38 Erw Goch**
**Ruthin Clwyd North Wales(GB)**

(72) Inventor: **Smith, Edward Sydney**
**38, Erw Goch Ruthin**
**Clwyd, LL15 1RR(GB)**
Inventor: **Davies, John**
**71 Fforddpentre Mold**
**Clwyd, CH7 1UY(GB)**

(74) Representative: **Abrams, Michael John et al**
**HASELTINE LAKE & CO. Hazlitt House 28 Southampton Buildings Chancery Lane London WC2A 1AT(GB)**

## Description

This invention relates to a displacement sensor. Displacement sensors which come in many different forms, as e.g. disclosed in FR-A-2 353 268, are usually designed to give a quantitative measurement of the displacement being measured. Often however a quantitative output is not required and an indication that movement has occurred suffices.

GB-2 176 610A (published 31 December, 1986 but having an earlier priority date than the present application) discloses a respiration monitor (which is a particular form of displacement sensor) and which comprises a permanent magnet mounted resiliently relative to a Hall effect device which detects changes in magnetic field due to movements of the magnet caused by a patient's breathing. The monitor includes a flexible mounting for the permanent magnet which mounting may take the form of a rubber or plastics diaphragm or a layer of a resilient rubber material. The monitor may be strapped by a belt or otherwise mounted on the patient's abdomen such that upon respiration the relative positions of the magnet and Hall effect device change, thus enabling the generation of an output signal from the sensor.

Document Proc. of the Nat. Electr. Conf., Vol. 33 (1979), Oct., pp. 280-284 discloses a medical displacement sensor utilizing relative changes of distance between a HALL generator and a movable magnet.

The present invention is set out in claim 1.

Further embodiments are contained in the dependent claims.

A specific embodiment of the invention will now be described by way of example with reference to the accompanying drawings, in which:-

FIGURE I shows in perspective a sensor having an electrical cord for connection to an electronic monitoring arrangement; and

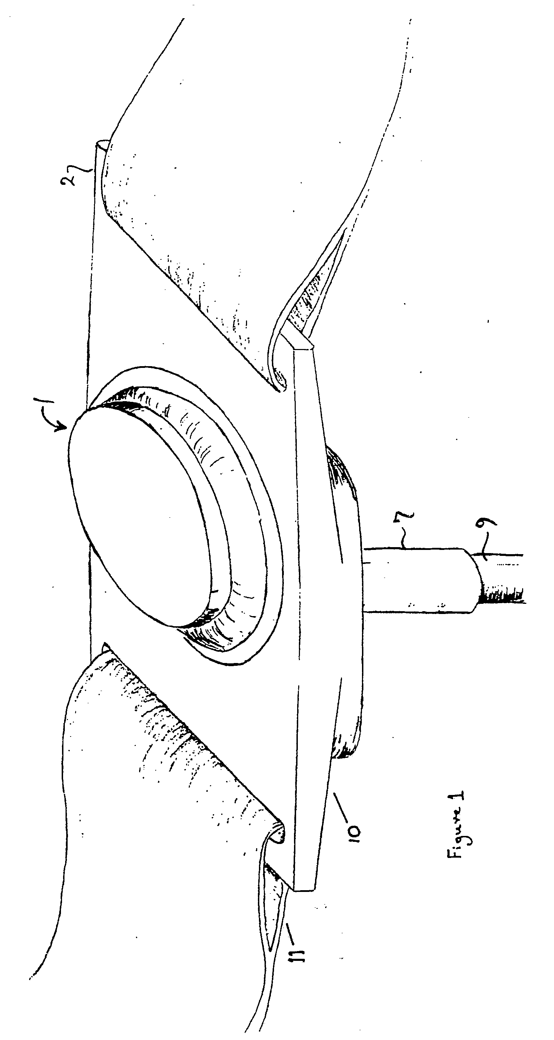

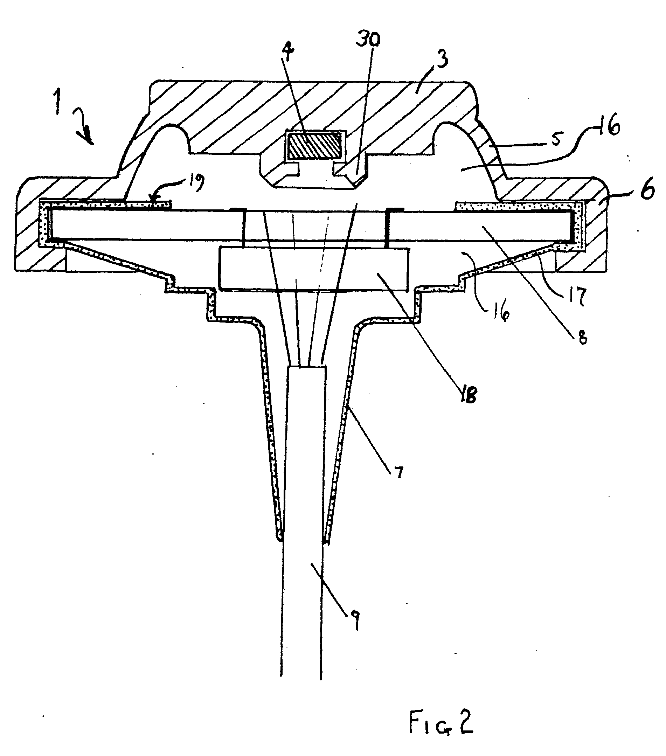

FIGURE 2 is a schematic cross-section through the sensor of Figure I indicating the method of mounting the Hall device and its associated magnet.

With reference to Figure I of the drawings, the displacement sensor (which is suitable for use inter. alia as a respiration monitor) comprises a housing I which fits into a plastics retaining plate 10 which has lateral extensions such as 2 for receiving a belt or similar fastening device 11. An electrically insulating sheathing 7 surrounds an output cable 9.

Referring now to Figure 2, the housing I comprises an upper, relatively thick walled cap portion 3 which includes a chamber in which is mounted a permanent magnet 4. An annular ring 30 projects downwardly from the inside of cap 3 in the region just below magnet 4. Ring 30 has a 'V'-shaped profile as shown. Downwardly and outwardly extending frustoconical wall portions 5 join cap portion 3 to a relatively thin walled portion 6 which defines an internal cavity or second chamber 16. Within this second chamber I6 there is located a printed circuit board 8 onto which a Hall effect device I8 is soldered.

Electrical connection to the sensor is effected by a four cored cable 9, soldered to the printed circuit board 8.

The sheathing 7 is in the form of a thin walled rubber shroud which fits tightly over the printed circuit board 8 and cable 9 and is sculptured to fit snugly over the Hall effect device I8 and to ensure that the sensor assembly sits securely in the plastic retaining plate I0.

The housing is formed of a resilient silicone rubber material and is arranged so that it clips over an extension I7 of sheathing 7 which in turn is folded over as at I9 to hold the printed circuit board 8. The whole assembly is then held by the plastics retaining plate I0 through which the retaining strap II is threaded, as shown in Figure I.

In usage the flat head of the cap 3 is held in contact against the surface to be monitored for movement. Movement causes deflection of the cap containing magnet 4 relative to the Hall effect device I8, thereby producing a change in the magnetic field coupling with the Hall device I8, and hence a change in its output.

The walls of the silicone rubber housing I, and in particular the cap 3 and wall portions 5, have a specially designed geometry as shown in Figure 2 and this leads to a particular characteristic such that the head of the cap 3 is compliant with the movement of the surface being monitored. This enables minute movements to be detected. The ability of the cap 3 to follow such movements is enhanced by the geometry of wall portion 6 and of sheathing 7 and I7 which, together with the structure of moulded plastics retaining plate I0, enables the cap 3 to retract into the cavity I6, but not so far such that its ability to track surface movement is impaired. This feature is enhanced for surfaces which come within the boundary of the retaining plate I0 by the resilient rubber ring 30 integral with the silicone cap 3 and having a profile in the form of a 'V' ring; this assists in preventing the magnet 4 from being held fixed to the surface on which it would otherwise foul.

The spacing between the Hall effect device I8 and the magnet 4 is preferably in the range 0.5 - 2.5mm, and preferably is of the order of Imm. While Figure 2 is not drawn exactly to scale, it nevertheless indicates approximate proportions and dimensions for the other components of a sensor in which the magnet-to-Hall effect device spacing is about Imm.

A sensor in accordance with this invention may be used in conjunction with an electrical circuit as disclosed in GB-2 176 610A.

## Claims

1. A displacement sensor which comprises a resilient housing (1) and a displacement transducer (18), wherein (a) said housing (1) incorporates a first chamber (15) and spaced therefrom a second chamber (16); (b) said displacement transducer (18) is a Hall effect device (18) which is mounted within one of said two chambers within said housing (1); (c) an activator (4) for said displacement transducer (18) is mounted within the other of said two chambers within said housing (1); and (d) an output arrangement (9) is provided for delivering a signal from the displacement transducer (18) when the sensor is actuated.

2. A displacement sensor as claimed in claim 1, characterised in that said housing (1) includes a relatively thick walled cap portion (3) incorporating said first chamber (15).

3. A displacement sensor as claimed in claim 2, characterised in that said housing includes a downwardly and outwardly extending frustoconical wall portion (5); depending from said cap portion (3) and, connected to said frustoconical wall portion (5), a relatively thin walled portion (6) defining said second chamber (16).

4. A displacement sensor as claimed in claim 3, characterised in that the Hall effect device (18) is mounted within said second chamber (16), and a permanent magnet (4) is mounted within said first chamber 15.

5. A displacement sensor as claimed in claim 1, 2, 3 or 4, further characterised by an electrical arrangement (8) in electrical contact with said Hall effect device and arranged to deliver an output signal via (9) when the sensor is subjected to movement such as to alter the relative position between said Hall effect device (18) and said permanent magnet (4).

6. A displacement sensor as claimed in any preceding claim, characterised in that said housing is supported on a retaining strap.

7. A displacement sensor as claimed in claim 2, 3 or 4, characterised in that said cap is formed with an inwardly projecting annular ring (30).

## Revendications

1. Détecteur de déplacement qui comporte un boîtier élastique (1) et un transducteur de déplacement (18), dans lequel (a) ledit boîtier (1) comprend une première chambre (15), et, espacée de celle-ci, une deuxième chambre (16); (b) ledit transducteur de déplacement (18) est un dispositif à effet Hall (18) qui est monté à l'intérieur de l'une desdites deux chambres à l'intérieur dudit boîtier (1); (c) un dispositif d'activation (4) pour ledit transducteur de déplacement (18) est monté à l'intérieur de l'autre desdites deux chambres à l'intérieur dudit boîtier; et (d) une disposition de sortie (9) est présente pour délivrer un signal venant du transducteur de déplacement (18) lorsque le détecteur est activé.

2. Détecteur de déplacement selon la revendication 1, caractérisé en ce que ledit boîtier (1) comporte une partie de capot (3) à paroi relativement épaisse comprenant ladite première chambre (15).

3. Détecteur de déplacement selon la revendication 2, caractérisé en ce que ledit boîtier comporte une partie de paroi (5) tronconique s'étendant vers le bas et vers l'extérieur; descendant depuis ladite partie de capot (3), et, connectée à ladite partie de paroi tronconique (5), une partie de paroi relativement fine (6) définissant ladite deuxième chambre (16).

4. Détecteur de déplacement selon la revendication 3, caractérisé en ce que le dispositif à effet Hall (18) est monté à l'intérieur de ladite deuxième chambre (16), et en ce qu'un aimant permanent (4) est monté à l'intérieur de ladite première chambre 15.

5. Détecteur de déplacement selon la revendication 1, 2, 3 ou 4, caractérisé de plus par une disposition électrique (8) en contact électrique avec ledit dispositif à effet Hall et conçue pour délivrer un signal de sortie par l'intermédiaire de (9) lorsque le détecteur est soumis à un déplacement visant à altérer la position relative entre ledit dispositif à effet Hall (18) et ledit aimant permanent (4).

6. Détecteur de déplacement selon l'une quelconque des revendications précédentes, caractérisé en ce que ledit boîtier est supporté sur une bande de maintien.

7. Détecteur de déplacement selon la revendication 2, 3 ou 4, caractérisé en ce que ledit capot est formé avec un anneau (30) s'étendant vers l'intérieur.

**Patentansprüche**

1. Ein Verschiebungssensor, der ein elastisches Gehäuse (1) und einen Verschiebungswandler (18) umfaßt, wobei (a) das Gehäuse (1) einen ersten Raum (15) und einen von diesem mit Abstand angeordneten zweiten Raum (16) enthält; (b) der Verschiebungswandler (18) eine Hall-Effekt-Einrichtung (18) ist, die innerhalb einer der beiden Räume innerhalb des Gehäuses (1) angebracht ist; (c) ein Aktivator (4) für den Verschiebungswandler (18) innerhalb des anderen der beiden Räume innerhalb des Gehäuses (1) angebracht ist; und (d) eine Ausgabe-Anordnung (9) vorgesehen ist zum Ausgeben eines Signals vom Verschiebungswandler (18), wenn der Sensor betätigt wird.

2. Ein Verschiebungssensor nach Anspruch 1, dadurch gekennzeichnet, daß das Gehäuse (1) einen relativ dickwandigen Verschlußteil (3) aufweist, der den ersten Raum (15) enthält.

3. Ein Verschiebungssensor nach Anspruch 2, dadurch gekennzeichnet, daß das Gehäuse einen sich nach unten und außen erstreckenden kegelstumpfförmigen Wandabschnitt (5), der am Verschlußteil (3) anhängt, und, verbunden mit diesem kegelstumpfförmigen Wandabschnitt (5), einen relativ dünnwandigen Abschnitt (6) umfaßt, der den zweiten Raum (16) abgrenzt.

4. Ein Verschiebungssensor nach Anspruch 3, dadurch gekennzeichnet, daß die Hall-Effekt-Einrichtung (18) innerhalb des zweiten Raums (16) angebracht ist und ein Permanentmagnet (4) innerhalb des ersten Raums (15) angebracht ist.

5. Ein Verschiebungssensor nach Anspruch 1, 2, 3 oder 4, ferner gekennzeichnet durch eine elektrische Anordnung (8), die in elektrischem Kontakt mit der Hall-Effekt-Einrichtung steht und so ausgebildet ist, daß sie über (9) ein Ausgangssignal ausgibt, wenn der Sensor einer Bewegung, wie z.B. zur Veränderung der relativen Stellung zwischen der Hall-Effekt-Einrichtung (18) und dem Permanentmagneten (4), unterworfen ist.

6. Ein Verschiebungssensor nach wenigstens einem der vorhergehenden Ansprüche, dadurch gekennzeichnet, daß das Gehäuse auf einem Halteband abgestützt ist.

7. Ein Verschiebungssensor nach Anspruch 2, 3 oder 4, dadurch gekennzeichnet, daß das Verschlußteil mit einem nach innen vorspringenden ringförmigen Ring (30) ausgebildet ist.

Figure 1

Fig 2